# EUROPEAN PATENT SPECIFICATION

(11) **EP 2 157 168 B1**
(45) Date of publication and mention of the grant of the patent: **11.11.2015**
(21) Application number: 08739888.9
(22) Date of filing: 04.04.2008
(51) Int. Cl.: C12N 1/20, A23K 1/16, A23K 1/18, C12N 15/09, C12P 23/00, C12R 1/01

(54) **ASTAXANTHIN-PRODUCING BACTERIUM, BACTERIAL CULTURE, ASTAXANTHIN-CONTAINING COMPOSITION AND METHOD OF PRODUCING ASTAXANTHIN**
ASTAXANTHIN PRODUZIERENDES BAKTERIUM, BAKTERIENKULTUR, ASTAXANTHIN ENTHAL-TENDE ZUSAMMENSETZUNG UND VERFAHREN ZUR HERSTELLUNG VON ASTAXANTHIN
BACTÉRIE DE PRODUCTION D'ASTAXANTHINE, DE CULTURE BACTÉRIENNE, COMPOSITION CONTENANT DE L'ASTAXANTHINE ET PROCÉDÉ DE PRODUCTION D'ASTAXANTHINE

(30) Priority: 12.04.2007 JP 2007104711
(43) Date of publication of application: 24.02.2010
(73) Proprietor: Electric Power Development Co., Ltd., Tokyo 104-8165 (JP)
(72) Inventor: MATSUMOTO, Mitsufumi, Kitakyusyu-shi Fukuoka 808-0111 (JP)
(74) Representative: Kling, Simone
(86) International application number: PCT/JP2008/056782
(87) International publication number: WO 2008/129934

(56) References cited:
- JP-A- 2006 191 919
- TAKAICHI S. ET AL.: "carotenoid sulfates from the aerobic photosynthetic bacterium, erythrobacter longus" PHYTOCHEMISTRY, vol. 30, no. 10, 1 January 1991 (1991-01-01), - 31 December 1991 (1991-12-31) pages 3411-3415, XP002578823
- KODERA K. ET AL.: 'Astaxanthin Gosei Kaiyo Bacterium no Screening to sono Characterization' JAPANESE SOCIETY FOR MARINE BIOTECHNOLOGY TAIKAI KOEN YOSHISHU vol. 7TH, 2004, page 117 + ABSTR. NO. AO-5, XP008142141
- YOON J.H. ET AL.: 'Erythrobacter aquimaris sp. nov., isolated from sea water of a tidal flat of the Yellow Sea in Korea' INT. J. SYST. EVOL. MICROBIOL. vol. 54, 2004, pages 1981 - 1985, XP008122062
- DATABASE GENBANK [Online] RAINEY F.A. ET AL.: 'Erythrobacter longus strain DSM 6997 16S ribosomal RNA gene, partial sequence', XP008115051 Retrieved from EMBL Database accession no. (AF465835)

## Description

### TECHNICAL FIELD

The present invention relates to a bacterium belonging to the genus *Erythrobacter* having an astaxanthin productivity, a bacterial culture product and an astaxanthin-containing composition produced by using said bacterium, and a method for producing astaxanthin using the bacterium.

Priority is claimed on Japanese Patent Application No.2007-104711, filed April 12, 2007, the content of which is incorporated herein by reference.

### BACKGROUND ART

Astaxanthin is a carotenoid pigment which is widely found in fish such as sea red bream and salmon, and crustaceans such as crab and shrimp.

In addition, the most famous example of producing microorganisms is a green alga *Heamatococcus pluvialis.*

Another example is a red yeast *Xanthophyllomyces dendrohous* (former *Phaffia rhodozyma*).

It is known that the amount of astaxanthin produced by *Heamatococcus pluvialis* is about 43 mg/g dry weight (Non-Patent Document 1).

It is also reported that the amount of astaxanthin produced by the red yeast *Xanthophyllomyces dendrohous* (former *Phaffia rhodozyma*) is 0.4 mg/g dry weight (Non-Patent Document 2).

Bacteria, which are prokaryotic cells, are one of the most expected microorganisms for the production of useful materials, since they have an extensive substrate utilization capacity, show a high growth rate under a basic culture, and have no thick cell wall unlike the yeast mentioned above.

In the astaxanthin production using bacteria, a gene recombinant of *Escherichia coli* has achieved the amount of 1.4 mg/g dry weight (Non-Patent Document 3).

In addition, a method using *Bacillus firmus* has achieved the amount of 0.05 mg/g dry weight (Non-Patent Document 4).

Furthermore, reportedly, a marine bacterium *Paracoccus sp.* MBIC1143 has achieved the amount of 0.14 mg/g dry weight (Non-Patent Document 5).

However, the astaxanthin production by these gene recombinants currently involves problems in terms of the expression level, for example.

On the other hand, in astaxanthin production by means of isolation from natural products, since astaxanthin is extracted from krill and crustaceans, the extraction efficiency is low and thus the extraction amount is small, which leads to a problem of cost increment.

In addition, it is also commercially problematic in terms of resource conservation due to a reduction of natural resources.

Astaxanthin has lots of physiological activities and is commercially available in the form of supplemental foods, for example.

Moreover, cultured fish such as sea bream and salmon are on an astaxanthin-containing diet (color enhancing feedstuffs) so that their colors can more closely resemble that of natural fish.

A recent reduction in natural resources has been expected for the production of natural resources by means of cultivation.

In the artificial seed production of fishery, there is a demand for feedstuffs to which high dietary effects and additional values have been added for the target fish species.

Fish species such as sea bream and rainbow trout, which are required to have bright body colors, are on an astaxanthin- or other pigment-containing diet.

Astaxanthin as an additive for color enhancing feedstuff is commercially available in the name of Calorie Pink (synthetic astaxanthin) from Roche. However, natural astaxanthin has been attracting an attention due to the issue of traceability and a preference for natural products.

So far, the green alga *Heamatococcus pluvialis* and the red yeast *Xanthophyllomyces dendrohous* have been used for color enhancing natural astaxanthin. However, there have been problems of insufficient coloration, for examples.

The green alga *Heamatococcus pluvialis* and the red yeast *Xanthophyllomyces dendrohous* have their own thick cell walls, because of which the rate of digestion/absorption in the target fish species is low.

For this reason, there is a demand for a new microorganism which has a high absorption rate.

Bacteria are characteristic in their thin cell walls and rapid proliferation. Therefore, astaxanthin-producing bacteria have been sought, and already known is the genus *Flavobacterium*, the genus *Alcaligenes*, the genus *Pseudomonas*, the genus *Alteromonas*, the genus *Hyphomonas*, the genus *Caryophanon*, the genus *Erythrobacter,* and the genus *Paracoccus.*
[Non-Patent Document 1] Lee, Y.-K. and Soh, C.-W.: Journal of Phycology (J. Phycol.), U.S.A., 1991, Vol. 27, No. 3, p.575-577
[Non-Patent Document 2] Flores-Cotera, L.B. et al.: Applied Microbiology and Biotechnology (Appl. Microbiol. Biotechnol.), U.S.A., 2001, Vol. 55, No. 2, p.341-347
[Non-Patent Document 3] Wang, C.-W. et al.: Biotechnology and Bioengineering (Biotechnol. Bioeng.), U.S.A., 1999, Vol. 62, No. 3, p.235-241
[Non-Patent Document 4] Yokoyama et al.: Bioscience, Biotechnology, and Biochemistry, U.S.A., 1994, Vol. 58, No.10, p.1842-1844
[Non-Patent Document 5] Pane, L. et al.: Journal of Biology Research (J. Biol. Res.), U.S.A., 1996, Vol. 22, p.303-308

### DISCLOSURE OF INVENTION

### PROBLEMS TO BE SOLVED BY THE INVENTION

It is an objective of the present invention to provide a bacterium which has no thick cell wall unlike yeasts and algae and is capable of producing astaxanthin, a bacterial culture product and an astaxanthin-containing composition produced by using said bacterium, and a method for producing astaxanthin using said bacterium.

### MEANS FOR SOLVING THE PROBLEMS

In order to solve the abovementioned problems, a first aspect of the present invention is a bacterium belonging to the genus *Erythrobacter* which can produce adonixanthin accounting for 35% by mass or more and astaxanthin accounting for 5% by mass or more, of the produced composition.

A second aspect of the present invention is a method for producing a bacterial culture product yielded by culturing any the abovementioned bacterium.

Another aspect of the present invention is a method for producing an astaxanthin-containing composition using said bacterium.

### EFFECTS OF THE INVENTION

With use of the novel bacterium of the present invention and the culture product thereof, it becomes possible to produce astaxanthin which serves as a useful pigment.

Furthermore, according to the astaxanthin-containing composition and the method for producing astaxanthin of the present invention, it becomes possible to efficiently produce astaxanthin which serves as a useful pigment, and to efficiently prepare feedstuffs such as color enhancing feedstuffs which contain more astaxanthin than conventional astaxanthin-containing feedstuffs.

### BRIEF DESCRIPTION OF THE DRAWINGS

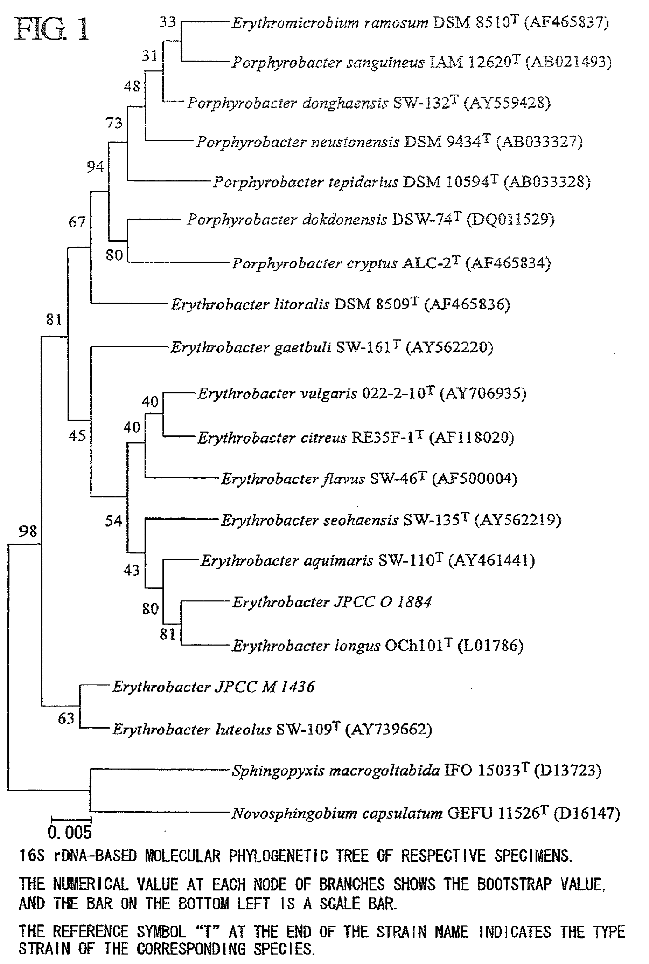

FIG. 1 is a molecular phylogenetic tree showing the result of molecular phylogenetic analysis of *Erythrobacter JPCC O sp*. strain 1884 (accession number NITE BP-341).

### BEST MODE FOR CARRYING OUT THE INVENTION

Hereunder is a detailed description of the present invention.

Hereinunder, the bacterium of "*Erythrobacter JPCC O sp.* strain 1884 (accession number NITE BP-341)" may be abbreviated as "*Erythrobacter JPCC O sp.* strain 1884" or *"JPCC O* 1884".

Moreover, in the present invention, the term astaxanthin may include its structural isomer (cis form).

In addition, the term "structural isomer" used herein refers to "cis form".

### <Acquisition of Erythrobacter JPCC O sp. strain 1884>

An attempt was made to acquire an astaxanthin-producing microorganism from sea sand, mud, water, and so forth collected from marine environments and mangrove forests.

100 µl of the sample was spread over an agar plate which was produced by adding yeast extract at 5.0 g/l, peptone at 1.0 g/l, and glucose at 5.0 g/l in artificial sea water (Senjyu Pharmaceutical).

Static culture was carried out at 25°C for 7 to 10 days to thereby acquire marine microorganisms forming orange and red colonies.

In order to isolate these microorganisms into single bacteria, the thus acquired marine microorganisms were inoculated in 5 ml of a liquid medium containing the abovementioned components, and were allowed to grow under shaking culture (150 rpm). The resultant solution was spread over an agar plate to form colonies. This procedure was repeated to effect isolation into single bacteria.

Pigments were extracted from 0.3 mg of a bacterial powder consisting of about 800 strains of marine bacteria by using a chloroform-methanol solution (1:1, v/v), and the screening of astaxanthin was performed.

The presence/absence of astaxanthin was assessed by TLC (thin layer chromatography).

As a result, strains producing astaxanthin and other various carotenoid pigments were identified, from which the *Erythrobacter JPCC O sp.* strain 1884 was acquired.

The *Erythrobacter JPCC O sp.* strain 1884 was deposited with the National Institute of Technology and Evaluation, Patent Microorganisms Depositary (2-5-8 Kazusakamatari, Kisarazu-shi, Chiba, Japan) as a national deposit as of March 19, 2007 (accession number: NITE BP-341 original deposit).

The request for transfer of the original deposit was accepted on February 29,2008 by the concerned authority under the Budapest Treaty, and the deposit was given the accession number of NITE BP-341.

### <Identification of Erythrobacter JPCC O sp. strain 1884>

The *Erythrobacter JPCC O sp*. strain 1884 was identified by TechnoSuruga Laboratory on consignment.

Then, the specimen acquired by the abovementioned method was subjected to validation of the "morphological properties", "culturing properties", "physiological properties", "acid/gas production from saccharides" and "other physiological properties", identification of the "nucleotide sequence", and phylogenetic analysis.

The "morphological properties" are shown in Table 1, the "culturing properties" are shown in Table 2, the "physiological properties" are shown in Table 3, the "acid/gas production from saccharides" is shown in Table 4, the "other physiological properties" are shown in Table 5, and the "16S rDNA sequence" is shown in SEQ ID NO:1.

In Table 1 to Table 5, (1) the items "gelatin liquefaction" and "gram staining property" were obtained with reference to the document "BARROW, (G.I.) and FELTHAM, (R.K.A): Cowan and Steel's Manual for the Identification of Medical Bacteria, 3rd edition, 1993, Cambridge University Press".

Moreover, (2) the items "culture condition with litmus milk", "MR test", "hydrolysis of starch", "utilization of citrate", "utilization of inorganic nitrogen source", "catalase", "oxidase", "O-F test (oxidation /fermentation)", and "acid/gas production from saccharides" were obtained with reference to the document "Toshikazu Sakazaki, Etsuro Yoshizaki, and Kanji Miki: Shin Saikin Baichigaku Kouza (The Course of Culturing Medium for Microorganism PART II), the latter volume, 2nd Edition, 1998, Kindai Shuppan, (Kinki University Press), Tokyo".

Furthermore, (3) the items of "nitrate reduction", "denitrification", "VP test", "indol production", "production of hydrogen sulfide", "urease activity", and "other physiological properties" were obtained by using the "bacterium identification kit API20E (bioMerieux, France)".

**[Table 1]**

| (Morphological properties) | |
|---|---|
| Culture condition | MB2216 agar medium at 25°C |
| Cell shape | Rod shape (0.7 - 0.8 x 1.0 - 3.0 µm) |
| Presence/absence of cell pleomorphism | + |
| Motility (adhesion status of flagellum) | - |
| Presence/absence of spore (site of spore) | - |

**[Table 2]**

| (Culturing properties) | |
|---|---|
| Culture condition | MB2216 agar medium at 25°C |
| Color | Orange |
| Glossiness | + |
| Pigment production | + |
| Culture condition | MB2216 agar medium at 25°C |
| Presence/absence of surface growth | - |
| Presence/absence of turbidity in medium | + |
| Culture condition | Gelatin stab culture at 25°C |
| Growth status | Presence of growth |
| Gelatin liquefaction ⁽¹⁾ | - |
| Culture condition ⁽²⁾ | Litmus milk at 25°C |
| Solidification | - |
| Liquefaction | - |

**[Table 3]**

| (Physiological properties) | | |
|---|---|---|
| Gram staining property ⁽¹⁾ | | - |
| Nitrate reduction ⁽³⁾ | | - |
| Denitrification ⁽³⁾ | | - |
| MR test ⁽²⁾ | | - |
| VP test ⁽³⁾ | | - |
| Indol production ⁽³⁾ | | - |
| Production of hydrogen sulfide ⁽³⁾ | | - |
| Hydrolysis of starch ⁽²⁾ | | - |
| Utilization of citrate ⁽²⁾ | (Koser) | - |
| | (Christensen) | - |
| Utilization of inorganic nitrogen source ⁽²⁾ | Nitrate salt | - |
| | Ammonium salt | - |
| Urease activity ⁽³⁾ | | - |
| Catalase ⁽²⁾ | | + |
| Oxidase ⁽²⁾ | | + |
| Range of growth | 6 | + |
| pH | 8 | + |
| | 9 | + |
| Range of growth | 15 | + |
| Temperature (°C) | 30 | + |
| | 37 | + |
| | 45 | - |
| Anaerobic viability | | - |
| O-F test (oxidation /fermentation) ⁽²⁾ | | -/- |

**[Table 4]**

| (Acid/gas production from saccharides ⁽²⁾) | |
|---|---|
| L-arabinose | -/- |
| D-glucose | -/- |
| D-fructose | -/- |
| Maltose | -/- |
| Lactose | -/- |
| D-sorbitol | -/- |
| Inositol | -/- |
| D-xylose | -/- |
| D-mannose | -/- |
| D-galactose | -/- |
| Sucrose | -/- |
| Trehalose | -/- |
| D-mannitol | -/- |
| Glycerin | -/- |

**[Table 5]**

| (Other physiological properties ⁽³⁾) | |
|---|---|
| β-galactosidase activity | - |
| Arginine dihydrolase activity | - |
| Lysine decarboxylase activity | - |
| Tryptophan deaminase activity | - |
| Gelatinase activity | - |

### (Nucleotide sequence)

The 16S rDNA of *Erythrobacter JPCC O sp.* strain 1884 (accession number NITE BP-341) was identified by a publicly known method, the result of which showed the nucleotide sequence represented by SEQ ID NO:1.

### (Phylogenetic analysis)

The analysis softwares used herein were the CLUSTAL W (refer to THOMPSON (J.D.), HIGGINS (D.G.) and GIBSON (T.J.), Nucleic Acids Research, 1994, 22:4673-4680), and the MEGA ver3.1 (refer to KUMAR, (S.), TAMURA, (K.), and NEI, (M.), Briefings in Bioinformatics, 2004, 5, 150-163). The obtained 16S rDNA sequence was subjected to molecular phylogenetic analysis by cross-checking with the nucleotide sequence information acquired from the bacteria type strain database, Apollon DB (TechnoSuruga Laboratory) and the International nucleotide sequence database (GenBank/DDBJ/EMBL).

The thus obtained molecular phylogenetic tree is shown in FIG. 1.

The obtained specimen is a bacillus belonging to α-proteobacteria, and is an obligate aerobic bacterium having a catalase and an oxidase.

As a result of the homology search with respect to the bacterium type strain database using BLAST (refer to ALTSCHUL, (S.F.), MADDEN, (T.F.), SCHAFFER, (A.A.), ZHANG, (J.), ZHANG, (Z.), MILLER, (W.), and LIPMAN, (D.J.), Nucleic Acids Research, 1997, 25: 3389-3402), the 16S rDNA sequence of the concerned specimen showed the highest homology of 97.8% with the 16S rDNA sequence of *Erythrobacter aquimaris* strain SW-110.

In addition, in the homology search with respect to the International nucleotide sequence database, the concerned specimen showed high homology with the 16S rDNA sequences derived from bacteria of *Erythrobacter.*

From these results, it was considered that the concerned specimen highly likely belongs to the genus *Erythrobacter.*

As a result of the molecular phylogenetic analysis, the concerned specimen forms a phylogenetic branch with *Erythrobacter longus*, which is a type species of *Erythrobacter,* and the bootstrap value of the phylogenetic branch between the concerned specimen and *Erythrobacter luteolus* was relatively high at 81%. The bootstrap value of a phylogenetic branch between these species and *Erythrobacter aquimaris* which is positioned outside of the phylogenetic branch between these species, was shown to be 80%. Therefore, the positions of these three strains were considered to be relatively stable.

From these results, the concerned specimen is considered to belong to the genus of *Erythrobacter* and closely related to known species of *Erythrobacter longus* and *Erythrobacter aquimaris.*

However, the 16S rDNA sequence of the concerned specimen is not completely homologous with the 16S rDNA sequences of these two species. Comparing the phylogenetic branches of the concerned specimen and these two species with the phylogenetic branches of other species belonging to the genus *Erythrobacter,* it can be considered that the concerned specimen is less likely to be the same species as any one of these two species.

From the abovementioned results of the molecular phylogenetic analysis, the concerned specimen was determined to be a novel species of the genus *Erythrobacter* (*Erythrobacter JPCC O sp.* strain 1884: accession number NITE BP-341).

### <Astaxanthin-producing bacterium, Bacterial culture product, Astaxanthin-containing composition, and Method for producing astaxanthin>

The bacterium of the present invention includes bacteria of *Erythrobacter JPCC O sp.* whose 16S rDNA sequence consists of the nucleotide sequence represented by SEQ ID NO: 1.

The reason is that such bacteria can be considered to be the same type of strain as the *Erythrobacter JPCC O sp.* strain 1884 (accession number NITE BP-341).

The bacteria of *Erythrobacter JPCC O sp.* whose 16S rDNA sequence consists of a nucleotide sequence having a 96% or higher homology with the nucleotide sequence represented by SEQ ID NO: 1 can include bacteria obtained by introducing mutation(s) into the 16S rDNA sequence of the bacterium of *Erythrobacter JPCC O sp.* strain 1884 (accession number NITE BP-341) by a publicly known method such as chemical treatments and gene recombination techniques.

As will be described later, bacteria which can produce large amounts of such pigments are of high utility value.

The method for producing astaxanthin of the present invention comprises the step of culturing the bacterium of the present invention mentioned above (hereunder, may be abbreviated as "the present bacterium").

The method for producing astaxanthin by using the present bacterium is exemplified as follows.

The medium for culturing the present bacterium is not specifically limited as long as it contains a carbon source, a nitrogen source, inorganic salts, trace elements (vitamins and trace metals), and other ingredients which are necessary for the growth of the present bacterium.

More specifically, examples of the carbon source can include saccharides such as glucose and sucrose, and alcohols such as ethanol and glycerol.

The dose may be roughly about 0.5 to 3.0% by mass, though it depends on the carbon source to be added.

The nitrogen source may be any of, for example, sodium nitrate, ammonium nitrate, ammonium sulfate, potassium nitrate, ammonium chloride, nitrate nitrogen such as urea, and ammonia nitrogen.

The dose may be roughly about 0.01 to 0.1% by mass, though it depends on the nitrogen source to be added.

As to the inorganic salts, there may be used, for example, sodium chloride, sodium sulphate, calcium chloride, potassium chloride, sodium hydrogencarbonate, potassium boride, strontium chloride, boric acid, sodium silicate, sodium fluoride, monopotassium phosphate, dipotassium phosphate, monosodium phosphate, disodium phosphate, iron chloride, manganese chloride, manganese sulfate, magnesium chloride, and copper sulfate.

The dose may be roughly about 0.001 to 0.01% by mass, though it depends on the inorganic salt to be added.

Moreover, as the special necessary ingredients, there may also be used yeast extract, peptone, triptone, and the like.

The dose of these special necessary ingredients may be roughly about 0.01 to 0.5% by mass, though it depends on the substance to be added.

In addition, the medium may also contain other ingredients to an extent that would not impair the effect of the present invention.

The medium for use in the culture of the present bacterium may be a commercial product, and the marine broth medium (manufactured by Difco) can be numerated as a preferable example.

Upon the culture of the present bacterium, the medium is preferably sterilized according to a publicly know method.

The pH of the medium is preferably adjusted at 6.0 to 8.0.

The culture temperature is preferably 20 to 35°C.

The culture duration is not specifically limited, although normally two to four days are preferred.

The culture method is preferably shaking culture or aeration culture.

In addition, the medium is preferably agitated during the culture.

The inoculation amount in the medium is not specifically limited, although it is preferably 0.1 to 5% by volume.

Next, astaxanthin is obtained from the cultured present bacterium.

For example, astaxanthin can be extracted with an organic solvent either directly from the bacterial culture product yielded by culturing the present bacterium, or from a precipitate recovered through centrifugation.

The bacterial culture product may also be subjected to the solvent extraction in a form of a dried bacterium made by lyophilization or other techniques.

The solvent used herein may be any solvent which can sufficiently dissolve astaxanthin. Such a solvent may be used either solely or in a form of a mixed solvent containing a plurality of types of solvents.

More specifically, a high polar solvent such as acetone, methanol, and ethyl acetate, and a non polar solvent such as hexane, dichloromethane, and chloroform may be used either solely or in a form of a mixture.

A mixed solvent containing chloroform and methanol is particularly preferred.

The mixture ratio thereof is not specifically limited, although it is preferably chloroform:methanol = 0.5:1.5 to 1.5:0.5 (v/v).

Furthermore, astaxanthin may also be purified by column chromatography using a filler such as silica gel and the solvent used for extraction or a similar solvent.

The components in the yielded composition can be identified by comparing its analytical data with the analytical data of well known compounds, for example.

Any analysis method may be employed as long as it can provide the structure-related data, and usual analysis methods such as HPLC, IR, NMR, and LC/MS may be employed.

Moreover, for example, the content of each component in the yielded composition can be quantified, if a standard curve is formed at the time of HPLC analysis.

The composition collected from the present bacterium also contains other various useful carotenoid pigments than astaxanthin, as components produced by the present bacterium in the course of conversion from β-carotene into astaxanthin.

Specific examples of pigments contained in the composition can include astaxanthin, adonixanthin, zeaxanthin, adonirubin, canthaxanthin, hydroxyechinenone, β-cryptoxanthin, echinenone, and β-carotene.

Of these, astaxanthin is contained at a high concentration of 5% by mass or more in the all produced pigments.

Moreover, astaxanthin produced by a bacterium having a usual astaxanthin productivity consists mainly of its trans form, whereas astaxanthin produced by the present bacterium includes its cis form at about 10% by mass or more.

Furthermore, adonixanthin is also contained at a particularly high concentration of 35% by mass or more.

Accordingly, the composition collected from the present bacterium is more useful than the composition produced by a bacterium having a usual astaxanthin productivity.

Astaxanthin can be also be produced by the following manner. The culture product yielded by culturing the present bacterium, alternatively a dried form, a frozen form, a solvent-extracted product, or a milled/crushed form of the present bacterium is added to a culture product of a zooplankton to be eaten, and the resultant matter is collected.

According to this production method, it also becomes possible to provide a feedstuff in which carotenoid pigments such as astaxanthin are concentrated.

Examples of such a zooplankton can include rotifer, artemia, and water flea.

The present bacterium can be concentrated in a biological manner by allowing these zooplanktons to eat the present bacterium. The thus resulting culture product of such a zooplankton is considerably concentrated as compared to astaxanthin and other carotenoid pigments produced by culturing conventional bacteria, and the utility value thereof is high.

The feedstuff produced by the method for producing astaxanthin or the like, which utilizes the concentration by a zooplankton can be suitably used for culturing fish.

It is known that some juvenile fish eat crustacean planktons containing carotenoid pigments which influence the color of fish.

Accordingly, upon the culture of these juvenile fish, if the feedstuff produced by the abovementioned method is supplied as a color enhancing feedstuff, it becomes possible to feed them with carotenoid pigments which have been concentrated by a zooplankton. Therefore, fish can be more efficiently colored than conventional methods.

### [Example]

Hereunder is a more detailed description of the present invention with a specific example.

However, the present invention is in no way limited to the following example.

### (Isolation of carotenoid pigments including astaxanthin from the culture product of Erythrobacter JPCC O sp. strain 1884)

The marine broth medium (manufactured by Difco) containing all necessary nutrients for the growth of *Erythrobacter JPCC O sp.* strain 1884 (accession number NITE BP-341) was prepared and sterilized at 121°C for 10 minutes.

Next, 250 ml of this medium was dispensed in a 1L Erlenmeyer flask, and was inoculated with 5 ml of this strain, followed by shaking culture at 150 rpm for three days.

This culture liquid was centrifuged and lyophilized to yield 0.5 g of a dried bacterial substance.

A chloroform-methanol solution (1:1, v/v) was added to this bacterial substance to extract pigments. Then, silica gel was resuspended in the mobile phase of chloroform-methanol and filled in an open column (diameter of 30 mm and total length of 600 mm). Then, pigments were separated using this column.

Pigments extracted from this strain were separated into five fractions.

Each fraction (fraction Nos. 1 to 5) was portioned out and analyzed by HPLC (column: diameter of 4.6 mm and total length of 250 mm). As a result, the presence of a substance showing the same vector as the publicly known vector of astaxanthin was confirmed in the fraction No. 2.

Furthermore, the analysis using LC/MS also provided the data consistent with the publicly known data of astaxanthin.

The other fractions (fraction Nos. 1, 3, 4, and 5) were also portioned out and analyzed by HPLC and LC/MS in the same manner as that of the fraction No. 2.

As a result, the presences of astaxanthin, adonixanthin, zeaxanthin, adonirubin, the structural isomer of astaxanthin, the structural isomer of adonixanthin, canthaxanthin, the structural isomer of hydroechinenone, β-cryptoxanthin, echinenone, the structural isomer of echinenone, β-carotene, and the structural isomer of β-carotene were confirmed.

The contents of major carotenoid pigments extracted from this strain were: astaxanthin at 0.114 mg/g, adonixanthin at 0.656 mg/g, zeaxanthin at 0.059 mg/g, adonirubin at 0.019 mg/g, the structural isomer of astaxanthin at 0.016 mg/g, the structural isomer of adonixanthin at 0.149 mg/g, canthaxanthin at 0.007 mg/g, the structural isomer of hydroechinenone at 0.065 mg/g, β-cryptoxanthin at 0.006 mg/g, echinenone at 0.165 mg/g, the structural isomer of echinenone at 0.001 mg/g, β-carotene at 0.064 mg/g, and the structural isomer of β-carotene at 0.001 mg/g, per gram of the dried bacterial substance. The total amount of these carotenoid pigments was 1.322 mg/g.

### INDUSTRIAL APPLICABILITY

The present invention can be used for the production of supplement foods and feedstuffs (color enhancing feedstuffs).

## Claims

1. A bacterium of *Erythrobacter JPCC O sp.* whose 16S rDNA sequence consists of the nucleotide sequence represented by SEQ ID NO:1 which can produce adonixanthin accounting for 35% by mass or more and astaxanthin accounting for 5% by mass or more, of the produced composition,
wherein the bacterium of *Erythrobacter JPCC O sp.* is a bacterium of *Erythrobacter JPCC O sp.* strain 1884 accession number: NITE BP-341.

2. A method for producing a bacterial culture product using a bacterium according to claim 1,
wherein the bacterium of *Erythrobacter JPCC O sp.* strain 1884 accession number NITE BP-341 in the bacterial culture product has a productivity capable of producing adonixanthin accounting for 35% by mass or more and astaxanthin accounting for 5% by mass or more of all produced pigments.

3. A method for producing an astaxanthin-containing composition using a bacterium according to claim 1,
wherein the bacterium of *Erythrobacter JPCC O sp.* strain 1884 accession number NITE BP-341 in the bacterial culture product has a productivity capable of producing adonixanthin accounting for 35% by mass or more and astaxanthin accounting for 5% by mass or more of all produced pigments.

## Patentansprüche

1. Bakterium von *Erythrobacter JPCC O sp.,* dessen 16S-rDNA-Sequenz aus der von SEQ ID NO:1 dargestellten Nukleotidsequenz besteht, das Adonixanthin produzieren kann, das 35 Massenprozent oder mehr der hergestellten Zusammensetzung ausmacht, und das Astaxanthin produzieren kann, das 5 Massenprozent oder mehr der produzierten Zusammensetzung ausmacht,
wobei das Bakterium von *Erythrobacter JPCC O sp.* ein Bakterium des *Erythrobacter-JPCC-O-sp.*-Stamms 1884, Hinterlegungsnummer: NITE BP-341, ist.

2. Verfahren zum Herstellen eines Bakterienkulturprodukts unter Verwendung eines Bakteriums nach Anspruch 1,
wobei das Bakterium des *Erythrobacter-JPCC-O-sp.*-Stamms 1884, Hinterlegungsnummer NITE BP-341, im Bakterienkulturprodukt eine Produktivität aufweist, die in der Lage ist, Adonixanthin zu produzieren, das 35 Massenprozent oder mehr aller produzierten Pigmente ausmacht, und Astaxanthin zu produzieren, das 5 Massenprozent oder mehr aller produzierten Pigmente ausmacht.

3. Verfahren zum Herstellen einer astaxanthinhaltigen Zusammensetzung unter Verwendung eines Bakteriums nach Anspruch 1,
wobei das Bakterium des *Erythrobacter-JPCC-O-sp.*-Stamms 1884, Hinterlegungsnummer NITE BP-341, im Bakterienkulturprodukt eine Produktivität aufweist, die in der Lage ist, Adonixanthin zu produzieren, das 35 Massenprozent oder mehr aller produzierten Pigmente ausmacht, und Astaxanthin zu produzieren, das 5 Massenprozent oder mehr aller produzierten Pigmente ausmacht.

## Revendications

1. Bactérie de l'espèce *Erythrobacter JPCC O* dont la séquence d'ADNr 16S consiste en la séquence de nucléotides représentée par SEQ ID NO: 1, qui peut produire de l'adonixanthine représentant 35 % en masse ou plus et de l'astaxanthine représentant 5 % en masse ou plus de la composition produite,
où la bactérie de l'espèce *Erythrobacter JPCC O* est une bactérie de l'espèce *Erythrobacter JPCC O* souche 1884 ayant le numéro d'accès NITE BP-341.

2. Procédé de production d'un produit de culture bactérienne en utilisant une bactérie selon la revendication 1,
dans lequel la bactérie de l'espèce *Erythrobacter JPCC O* souche 1884 ayant le numéro d'accès NITE BP-341 dans le produit de culture bactérienne présente une productivité capable de produire de l'adonixanthine représentant 35 % en masse ou plus et de l'astaxanthine représentant 5 % en masse ou plus de tous les pigments produits.

3. Procédé de production d'une composition contenant de l'astaxanthine en utilisant une bactérie selon la revendication 1,
dans lequel la bactérie de l'espèce *Erythrobacter JPCC O* souche 1884 ayant le numéro d'accès NITE BP-341 dans le produit de culture bactérienne présente une productivité capable de produire de l'adonixanthine représentant 35 % en masse ou plus et de l'astaxanthine représentant 5 % en masse ou plus de tous les pigments produits.


### SEQUENCE LISTING

<110>Electric Power Development Co.,Ttd
<120>Astaxanthin producing bacterium, its product and method for producing astaxanthin.
<130>OSP-30376
<150>JP 2007-104711
   <151> 2007-4-12
<160>1
<210>1
   <211>1406
   <212>DNA
   <213>ErythrobacterJPCC O 1884
<400>1
   tcagaacgaa cgctggcggc atgcctaaca catgcaagtc gaacgaaccc ttcggggtga 60
   gtggcgcacg ggtgcgtaac gcgtgggaac ctgcctttag gttcggaata actcagagaa 120
   atttgagcta ataccggata atgtcttcgg accaaagatt tatcgccttt agatgggccc 180
   gcgttagatt agatagttgg tggggtaatg gcctaccaag tcgacgatct atagctggtc 240
   tgagaggatg atcagccaca ctgggactga gacacggccc agactcctac gggaggcagc 300
   agtggggaat attggacaat gcgggaaacc gtgatccagc aatgccgcgt gagtgatgaa 360
   ggccttaggg ttgtaaagct cttttactag ggatgataat gacagtacct agagaataag 420
   ctccggctaa ctccgtgcca gcagccgcgg taatacggag ggagctagcg ttgttcggaa 480
   ttactgggcg taaagcgcgc gtaggcggct attcaagtca gaggtgaaat cccagggctc 540
   aaccctggaa ctgcctttga aactagatag ctagaatact ggagaggtga gtggaattcc 600
   gagtgtagag gtgaaattcg tagatattcg gaagaacacc agtggcgaag gcgactcact 660
   ggacagttat tgacgctgag gtgcgaaagc gtggggagca aacaggatta gataccctgg 720
   tagtccacgc cgtaaacgat gataactagc tgtccgggct catagagctt gggtggcgca 780
   gctaacgcat taagttatcc gcctggggag tacggtcgca agattaaaac tcaaaggaat 840
   tgacgggggc ctgcacaagc ggtggagcat gtggtttaat tcgaagcaac gcgcagaacc 900
   ttaccagcct ttgacatcct aggacggttt ctggagacag actccttccc ttcggggacc 960
   tagtgacagg tgctgcatgg ctgtcgtcag ctcgtgtcgt gagatgttgg gttaagtccc 1020
   gcaacgagcg caaccctcat ccttagttgc catcatttag ttgggcactt taaggaaact 1080
   gccggtgata agccggagga aggtggggat gacgtcaagt cctcatggcc cttacaggct 1140
   gggctacaca cgtgctacaa tggtatctac agtgggcagc gaacacgcga gtgtaagcta 1200
   atctccaaaa gatatctcag ttcggattgt tctctgcaac tcgagagcat gaaggcggaa 1260
   tcgctagtaa tcgcggatca gcatgccgcg gtgaatacgt tcccaggcct tgtacacacc 1320
   gcccgtcaca ccatgggagt tggtttcacc cgaagatagt gcgctaacct tttaggaggc 1380
   agctagccac ggtgggatca gcgact 1406